# EUROPEAN PATENT SPECIFICATION

(11) **EP 1 556 104 B1**
(45) Date of publication and mention of the grant of the patent: **02.04.2008**
(21) Application number: 03776241.6
(22) Date of filing: 07.10.2003
(51) Int. Cl.: A61M 5/142

(54) **MEDICAL CASSETTE PUMP WITH SINGLE FORCE SENSOR TO DETERMINE THE OPERATING STATUS**
MEDIZINISCHE KASSETTENPUMPE MIT EINZELNEM KRAFTSENSOR ZUR BESTIMMUNG DES BERIEBSSTATUS
POMPE MEDICALE D'UNE CASSETTE A CAPTEUR DE FORCE UNIQUE PERMETTANT DE DETERMINER L'ETAT DU FONCTIONNEMENT

(30) Priority: 16.10.2002 US 418914 P; 16.10.2002 US 418986 P; 22.07.2003 US 624667; 22.07.2003 US 624578
(43) Date of publication of application: 27.07.2005
(73) Proprietor: ABBOTT LABORATORIES, Abbott Park, IL 60064-6008 (US)
(72) Inventor: BOUTON, Chad, E., Delaware, OH 43015 (US); RADCLIFF, Dale, M., Hilliard, OH 43026 (US); NELSON, Steven, R., Grove City, OH 43123 (US); FORTNEY, Clark, E., Gahanna, OH 43230 (US); SMITH, Roger, W., Grove City, OH 43123 (US)
(74) Representative: Modiano, Micaela Nadia
(86) International application number: PCT/US2003/031703
(87) International publication number: WO 2004/035116

(56) References cited:
- US-A- 4 453 931
- US-A- 4 927 411
- US-A- 5 056 992
- US-A- 5 464 392
- US-A- 5 554 013

## Description

### BACKGROUND OF THE INVENTION

The present invention relates to a means of determining the operating condition of a medical pump. More particularly, this invention relates to a means of determining fluid status in positive displacement fluid pumping devices for the delivery of fluids to a patient.

Modern medical care often involves the use of medical pump devices to deliver fluids and/or fluid medicine to patients. Medical pumps permit the controlled delivery of fluids to a patient, and such pumps have largely replaced gravity flow systems, primarily due to the pump's much greater accuracy in delivery rates and dosages, and due to the possibility for flexible yet controlled delivery schedules. Of the modern medical pumps, those incorporating a diaphragm or pump cassette are often preferred because they provide a more accurate controlled rate and volume than do other types of pumps.

A typical positive displacement pump system includes a pump device driver and a disposable cassette. The disposable cassette, which is adapted to be used only for a single patient and for one fluid delivery cycle, is typically a small plastic unit having an inlet and an outlet respectively connected through flexible tubing to the fluid supply container and to the patient receiving the fluid. The cassette includes a pumping chamber, with the flow of fluid through the chamber being controlled by a plunger or pumping element 38 activated in a controlled manner by the device driver.

For example, the cassette chamber may have one wall formed by a flexible diaphragm which is reciprocated by the plunger and the driver to cause fluid to flow. The pump driver device includes the plunger or pumping element 38 for controlling the flow of fluid into and out of the pumping chamber in the cassette, and it also includes control mechanisms to assure that the fluid is delivered to the patient at a pre-set rate, in a pre-determined manner, and only for a particular pre-selected time or total dosage.

The fluid enters the cassette through an inlet and is forced through an outlet under pressure. The fluid is delivered to the outlet when the pump plunger forces the membrane into the pumping chamber to displace the fluid. During the intake stroke the pump plunger draws back, the membrane covering the pumping chamber pulls back from its prior fully displaced configuration, and the fluid is then drawn through the open inlet and into the pumping chamber. In a pumping stroke, the pump plunger forces the membrane back into the pumping chamber to force the fluid contained therein through the outlet. Thus, the fluid flows from the cassette in a series of spaced-apart pulses rather than in a continuous flow.

One of the requirements for a medical pump is that it is able to detect when it is operating under certain abnormal situations and to alert the user to these problems. Specifically, the pump should detect when flow of fluid is blocked, there is no fluid in the line, there is no cassette in the pump, if the pump has primed correctly, and if the valves in the pump are sealing properly.

Previous pumps that could supply all this information used at least two sensors associated with the pumping chamber or tubes to provide input regarding the fluid conditions to the control system. The use of multiple sensors requires more physical space on the pump and potentially results in a higher unit manufacturing cost.

Examples of sensor arrangements in medical pumping devices, that are suitable to monitor pressure are available from US-A- 4927411, US-A-4453931 and US-A-5464392.

It is therefore a principal object of this invention to provide means for using a single pressure sensor to discriminate between operating conditions in a medical pump.

These and other objects will be apparent to those skilled in the art.

### SUMMARY OF THE INVENTION

A medical pump, for use with a cassette having a pumping chamber, is disclosed, having the features set forth in Claim 1. The pump includes a pumping element with a piston slider assembly which intermittently pressurizes the pumping chamber during a pumping cycle. The piston slider assembly has a piston head connected to a main body with a single pressure sensor positioned therebetween. A camshaft is associated with the pumping element, an inlet control element, and an outlet control element for closing the pumping chamber to flow when the pumping chamber is pressurized. A position sensor detects the position of the pumping element. A processing unit receives pressure and position data from the pressure and position sensors. The processing unit processes this data to determine the operating condition of the pump. The operating conditions determined include: blocked fluid flow, no fluid in the line, no cassette associated with the pump, proper pump priming, or proper valve sealing.

### DESCRIPTION OF THE DRAWINGS

Fig. 1 is a schematic diagram of the cassette pump, illustrating the functional components of the pump and the cassette;
Figs. 2 and 3 are perspective views of a cassette for use with the pump of the present invention;
Fig. 4 is an internal side view of the pump of the present invention;
Fig. 5 is a perspective view of the pumping element of the present invention;
Fig. 6A is a perspective view of the inlet control element of the present invention;
Fig. 6B is a perspective view of the outlet control element of the present invention;
Fig. 7 is a side view of the camshaft of the present invention;
Fig. 8 is a sectional view taken along line A-A of Fig. 7, showing the piston lobe of the camshaft of the present invention;
Fig. 9 is a sectional view taken along line B-B of Fig. 7, showing the inlet lobe of the camshaft of the present invention;
Fig. 10 is a sectional view taken along line C-C of Fig. 7, showing the outlet lobe of the camshaft of the present invention;
Fig. 11 is an end view of sensor magnets located on the camshaft of the pump of the present invention;
Fig. 12 is a graph showing force data from a pump cycle illustrating an abnormal air stroke;
Fig. 13 is a graph showing force data from a pump cycle illustrating a normal fluid stroke;
Fig. 14 is a graph showing force data illustrating the presence and absence of a cassette in the pump;
Fig. 15 is a graph showing force data from a pump cycle illustrating impeded outlet flow; and
Fig. 16 is a graph showing flow rate test data taken with a wide range of cassettes and pumps employing the present invention.

### DETAILED DESCRIPTION OF THE PREFERRED EMBODIMENT

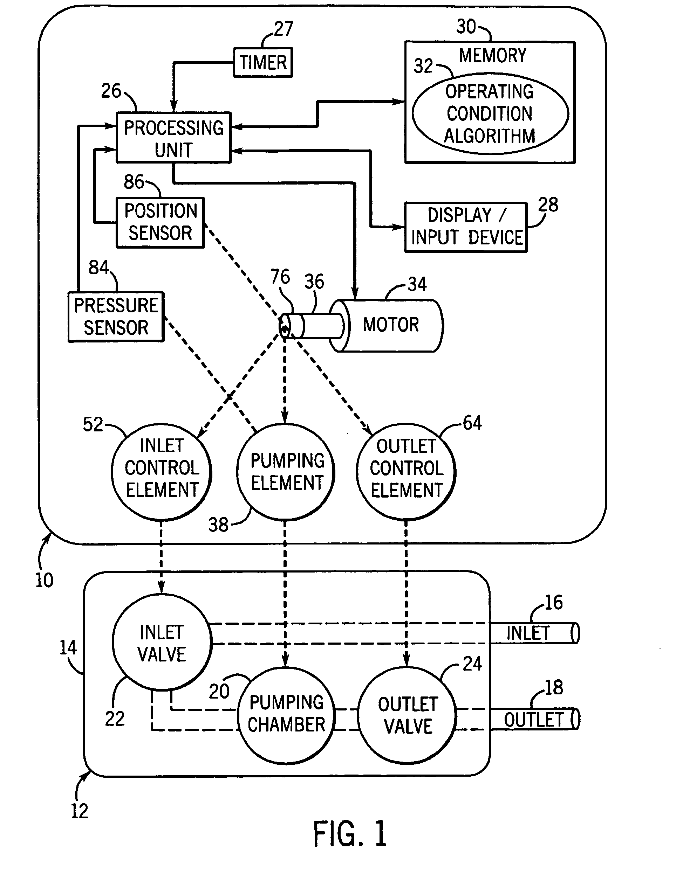

Fig. 1 is a schematic diagram illustrating the functional components of a medical pump 10 which is used in connection with a disposable cassette 12 for delivering a fluid to a patient. It will be understood to one of ordinary skill in the art that the term medical pump as used herein includes but is not limited to enteral pumps, parenteral infusion pumps, ambulatory pumps, or any positive displacement fluid pumping device for the delivery of fluids to a patient.

The medical pump 10 and cassette 12 are shown with several components for implementing the present invention. Those of ordinary skill in the art will appreciate that the pump 10 and cassette 12 include many more components than those shown in Fig. 1. However, it is not necessary that all these components be shown in order to disclose an illustrative embodiment for practicing the present invention. Commonly assigned and co-pending non-provisional application U.S. Serial No. 29/166,389 entitled PUMP CASSETTE discloses the particular cassette 12 described below. Pump cassettes and cassette pumps in general are well known in the art of medical fluid delivery, as evidenced by commonly assigned U.S. Patent Nos. 4,818,186; 4,842,584; and 5,000,664, the entire disclosure and drawings of which are hereby specifically incorporated herein by reference.

Cassette 12 includes a housing 14 on which is disposed an inlet port 16 for accepting the fluid flowing from an IV bag or other fluid container (not shown). Similarly, fluid lines (not shown) couple an outlet port 18 on housing 14 to the body of a patient.

A pumping chamber 20 is connected in fluid flow communication between the inlet port 16 and the outlet port 18. The pumping chamber 20 operates to meter fluid through the cassette 12.

An inlet valve 22 resides between inlet port 16 and the pumping chamber 20. Inlet valve 22 operates to physically open and close the fluid communication between inlet port 16 and pumping chamber 20.

Similarly, an outlet valve 24 resides between the pumping chamber 20 and outlet port 18. Outlet valve 24 operates to physically open and close the fluid communication between pumping chamber 20 and outlet port 18. The pumping chamber 20, inlet valve 22, and outlet valve 24 are all operatively associated with the pump 10 to control the flow of fluid through the cassette 12.

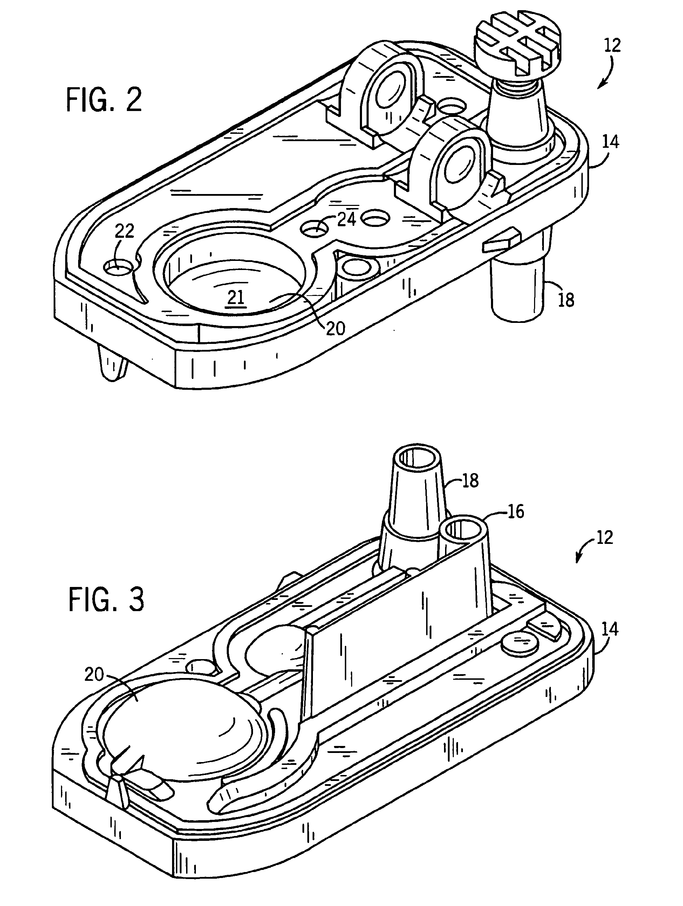

Referring to Figs. 2 and 3, one embodiment of cassette 12 is shown. It will be understood to one of ordinary skill in the art that a cassette having a different design than that shown in Figs. 2-3 may be used with pump 10 without departing from the present invention.

Referring to Fig. 1, a processing unit 26 is included in pump 10 and performs various operations described in greater detail below. A display/input device 28 communicates with the processing unit 26 and allows the user to receive output from processing unit 26 and/or input into the processing unit 26. Those of ordinary skill in the art will appreciate that display/input device 28 may be provided as a separate display device and a separate input device.

A memory 30 communicates with the processing unit 26 and stores code and data necessary for the processing unit 26 to calculate and output the operating conditions of pump 10. More specifically, the memory 30 stores an algorithm code 32 formed in accordance with the present invention for processing data to determine the operating condition of the pump 10.

These algorithms, Figure of Merit calculations and other details of the method for using pressure and position data to determine the operating condition of the pump 10 not discussed herein can be determined by reference to commonly assigned and co-pending non-provisional application entitled METHOD FOR DISCRIMINATING BETWEEN OPERATING CONDITIONS IN MEDICAL PUMP, which claims priority from provisional applications U.S. Ser. No. 60/418,914 and 60/418,986, the disclosure and drawings of which are hereby specifically incorporated herein by reference in its entirety. The disclosures and drawings of the provisional applications U.S. Ser. No. 60/418,986 and 60/418,914 are also specifically incorporated herein by reference in their entirety.

An electric motor 34 is controlled by processing unit 26 is energized by a power supply (not shown) to serve as a prime mover for rotatably driving a shaft 36. The motor 34 is a 6-volt, permanent magnet, DC gear motor with a 249:1 gear (not shown) on the output of the motor shaft 36. This motor 34 runs at different speeds depending on flow rates. The down-stroke or delivery portion of the stroke has the motor 34 running directly from power supply (not shown). The up-stroke, retract or fill portion of the stroke is run at a voltage set by the processing unit 26, so that the retract times are approximately 1.3, 1.4, 1.6, or 2.0 seconds, where higher flow rates require faster retract speeds.

A pumping element 38 is operatively associated with the shaft 36. When energized, the pumping element 38 reciprocates back and forth to periodically down-stroke, causing pumping element 38 to press on the diaphragm 21 of pumping chamber 20, driving fluid through cassette 12. On an up-stroke, pumping element 38 releases pressure from pumping chamber 20 and thereby drawing fluid from inlet port 16 into pumping chamber 20.

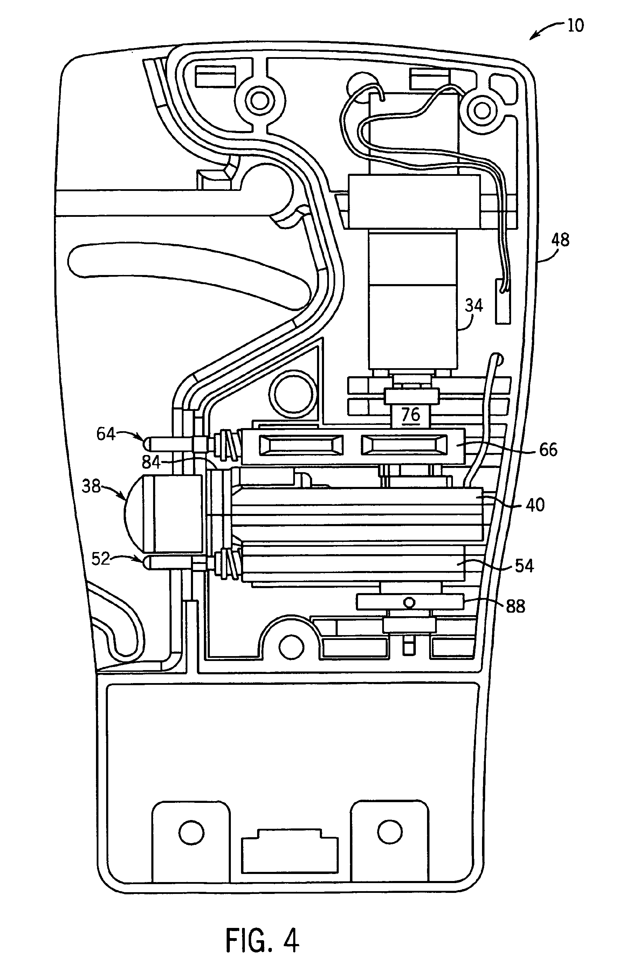

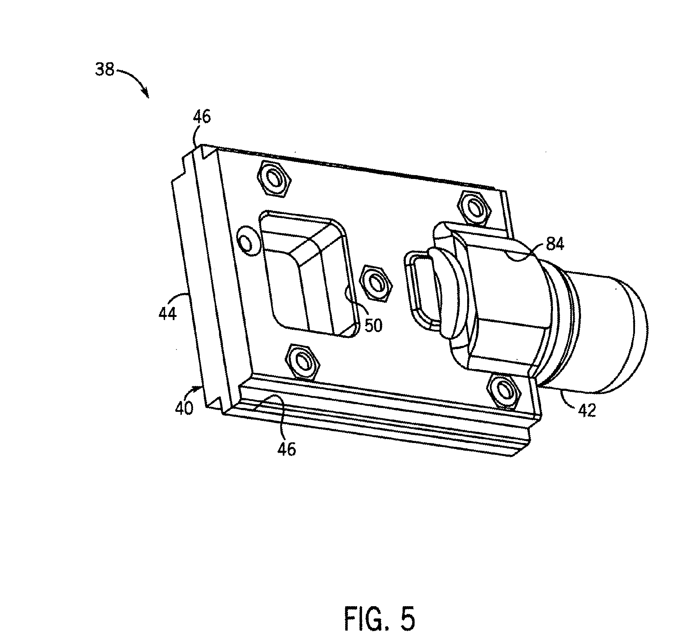

Referring to Figs. 4 and 5, the pumping element 38 is formed as a piston slider assembly 40. The piston slider assembly 40 includes a piston head 42 for contacting the pumping chamber 20 connected to a main body 44. Sliders 46 permit the piston slider assembly 40 to be slidably associated with rails (not shown) within a pump housing 48. A bore 50 passes through main body 44 and provides a surface for transferring force from the motor 34 to the pumping element 38.

Referring to Fig. 1, an inlet control element 52 is operatively associated with the shaft 36. When energized, inlet control element 52 reciprocates back and forth to periodically down-stroke, causing inlet control element 52 to press on inlet valve 22, closing pumping chamber 20 to fluid influx. On an up-stroke, inlet control element 52 releases pressure from inlet valve 22 and thereby allows the flow of fluid from inlet port 16 into pumping chamber 20.

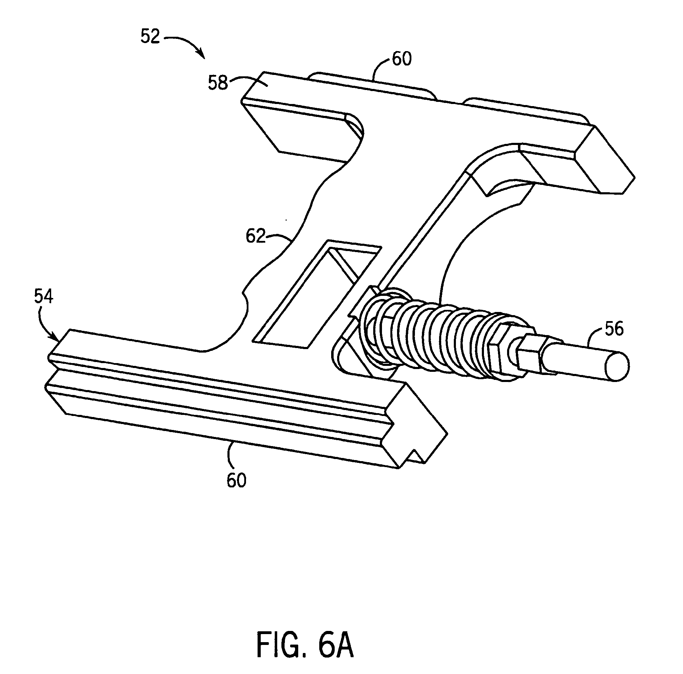

Referring to Figs. 4 and 6A, the inlet control element 52 is formed as an inlet slider assembly 54. The inlet slider assembly 54 includes an inlet pin 56 for contacting the inlet valve 22 and is connected to and spring biased against a main body 58. Sliders 60 permit the inlet slider assembly 54 to be slidably associated with rails (not shown) within pump housing 48. A notch 62 is positioned on the main body 58 to provide a surface for transferring force from the motor 34 to the inlet control element 52.

Referring to Fig. 1, an outlet control element 64 is operatively associated with the shaft 36. When energized, outlet control element 64 reciprocates back and forth to periodically down-stroke, causing outlet control element 64 to press on outlet valve 24, closing pumping chamber 20 to fluid efflux. On an up-stroke, outlet control element 64 releases pressure from outlet valve 24 and thereby allows the flow of fluid from pumping chamber 20 to outlet port 18. Thus the open or closed state of pumping chamber 20 is controlled by the positioning and movement of inlet and outlet control elements 52 and 64.

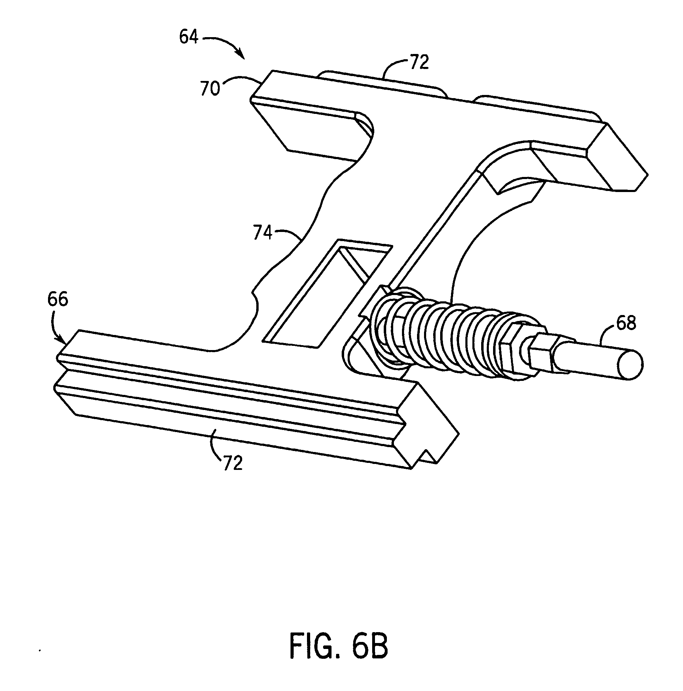

Referring to Figs. 4 and 6B, the outlet control element 64 is formed as an outlet slider assembly 66. The outlet slider assembly 66 includes an outlet pin 68 for contacting the outlet valve 24 and is connected to and spring biased against a main body 70. Sliders 72 permit the outlet slider assembly 66 to be slidably associated with rails (not shown) within pump housing 48. A notch 74 is positioned on the main body 70 to provide a surface for transferring force from the motor 34 to the outlet control element 64.

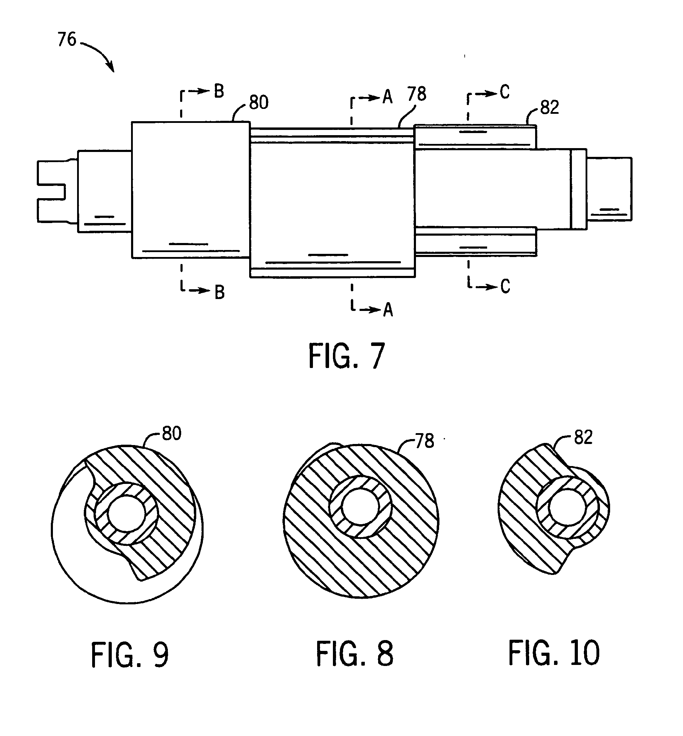

Referring to Figs. 1 and 7-10, a one-piece camshaft 76 is connected to shaft 36 and is thereby driven by the motor 34. The camshaft 76 has three lobes, with one lobe to drive the pumping element 38, inlet control element 52, and outlet control element 64. The camshaft 76 insures that the inlet and outlet valves 22 and 24 always will open and close at the appropriate times relative to the pumping element 38 stroke to achieve correct pump 10 operation.

A piston lobe 78 of the camshaft 76 is received within bore 50 of piston slider assembly 40. During rotation of camshaft 76, the piston lobe 78 pushes against bore 50 to drive piston slider assembly 40 back and forth within the pump housing 48. These movements result in piston head 42 intermittently pushing the pumping chamber 20 inward to drive fluid through cassette 12.

An inlet lobe 80 of the camshaft 76 contacts notch 62 of inlet slider assembly 54. During rotation of camshaft 76, the inlet lobe 80 pushes against notch 62 to drive inlet slider assembly 54 forward within the pump housing 48. These movements result in inlet pin 56 intermittently being compressed against the inlet valve 22 to cut off pumping chamber 20 from influent. Once the inlet lobe 80 releases this pressure on inlet pin 56, the spring biased compression force in inlet pin 56 is released against the main body 58, causing the main body 58 to retract from the inlet valve 22.

An outlet lobe 82 of the camshaft 76 contacts notch 74 of outlet slider assembly 66. During rotation of camshaft 76, the outlet lobe 82 pushes against notch 74 to drive outlet slider assembly 66 forward within the pump housing 48. These movements result in inlet pin 68 intermittently being compressed against the outlet valve 24 to cut off pumping chamber 20 from discharging effluent. Once the outlet lobe 82 releases this pressure on outlet pin 68, the spring biased compression force in outlet pin 68 is released against the main body 70, causing the main body 70 to retract from the outlet valve 24.

It will be understood by those skilled in the art, that the notches 62 and 74 and inlet and outlet lobes 80 and 82 associated therewith may be oriented and arranged so that the inlet and outlet lobes 80 and 82 move the inlet and outlet slider assemblies 54 and 56 back from cassette 12 without the assistance of spring biased pins 56 and 68.

The inlet and outlet lobes 80 and 82 operate to open and close inlet and outlet valves 22 and 24 at appropriate times to assure that there is unidirectional flow of the feeding liquid through the cassette 12. Because one or the other of the inlet and outlet slider assemblies 54 and 66 is in the closed position at any given point in the pumping element 38 stroke, they also prevent free flow of liquid through the cassette 12. In addition to preventing free flow, the inlet and outlet lobes 80 and 82 are designed to keep both of the inlet and outlet slider assemblies 54 and 66 in the closed position simultaneously during the first 35° of the pumping cycle, which permits relevant pressure data to be taken.

Referring to Fig. 1, a pressure sensor 84 is operatively associated with the pumping element 38. The pressure sensor 84 senses the force on pumping element 38 and generates a pressure signal based on this force. The pressure sensor 84 communicates with the processing unit 26, sending the pressure signal to the processing unit 26 for use in determining operating conditions of pump 10.

One of ordinary skill in the art will appreciate that the pressure sensor 84 may be a force transducer or any other device that can operatively sense the force brought to bear on the pumping chamber 20 by pumping element 38. The sensor 84 measures the force, which the pumping element 38 is pressing on the diaphragm 21. This force consists of two major components; the force required to displace the diaphragm 21 and the pressure of the fluid in the pumping chamber 20.

Referring to Figs. 1, 4 and 5, the pressure sensor 84 is attached to the piston slider assembly 40. The pressure sensor 84 is connected directly between the piston head 42 and the main body 44. As the piston slider assembly 40 is brought to bear on the pumping chamber 20, the piston head 42 presses against pressure sensor 84. Pressure sensor 84 senses this force and generates a pressure signal to the processing unit 26.

Referring to Fig. 1, a position sensor 86 tracks the pumping cycle of pump 10 by determining the position of the pumping element 38. The position sensor 86 is shown as operatively associated with the shaft 36. The position sensor 86 generates a position signal by directly or indirectly detecting the position of the pumping element 38. The position signal is sent to the processing unit 26. The processing unit 26 utilizes this information to associate the incoming pressure data with a particular portion of the pump cycle. One of ordinary skill in the art will appreciate that the position sensor 86 as used herein includes but is not limited to mechanical indicators such as pivoting dial indicators, electronic switches, Hall Effect sensors, and optical based position detectors.

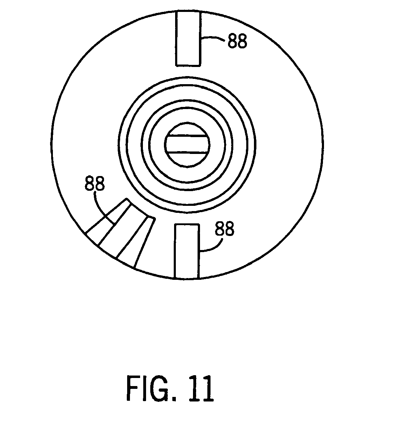

Referring to Figs. 1, 4 and 11, the position sensor 86 is a Hall Effect sensor having magnets 88 in relational contact with shaft 36. The Hall Effect sensor 86 monitors the magnets 88 to determine the rotational position of shaft 36. The rotational position of shaft 36 is used to indirectly detect the position of the pumping element 38. The position sensor 86 communicates with the processing unit 26, sending the position signal to the processing unit 26 for use in determining operating conditions of pump 10. One of ordinary skill in the art will appreciate that the position sensor 86 can track the shaft 36, a camshaft 76 attached to the shaft 36, or the pumping element 38 itself.

Referring to Fig. 1, in operation, at the beginning of a pumping cycle, outlet control element 64 operates to close outlet valve 24 so that there is no fluid communication between pumping chamber 20 and outlet port 18. Inlet valve 22 is opened to permit pumping chamber 20 to be in fluid communication with inlet port 16. In the next phase of the pumping cycle, inlet control element 52 operates to close inlet valve 22, thereby closing fluid communication between inlet port 16 and pumping chamber 20. Outlet valve 24 continues to remain closed. Next, pumping element 38 begins a down-stroke movement which presses pumping element 38 against pumping chamber 20, causing pumping chamber 20 to compress, thereby increasing the pressure within pumping chamber 20. Pressure sensor 84 reads and transmits this pressure data to processing unit 26. Under normal conditions the pumping chamber 20 is compressed sufficiently and a desired pressure profile is generated. At a given position of the shaft 36, the outlet control element 64 operates to open outlet valve 24 so that fluid flows from pumping chamber 20 to outlet port 18. The pump cycle then repeats.

The processing unit 26 retrieves the operating condition algorithm 32 from memory 30 and applies it to the pressure and position data received from this pump cycle. The pump pressure data and pump position data are processed. Sensing the force that the pumping chamber 20 exerts against the pumping element 38 and comparing that force to what one would expect to sense at that point in the cycle can determine all of the following operating conditions: when flow of fluid is blocked (occlusion), there is no fluid in the line, there is no cassette in the pump, if the pump has primed correctly, and if the valve in the pump are sealing properly. Once the operating condition is determined, the processing unit 26 outputs the operating condition on the display 28 and/or uses the determined operating condition to adjust operation of the pump 10.

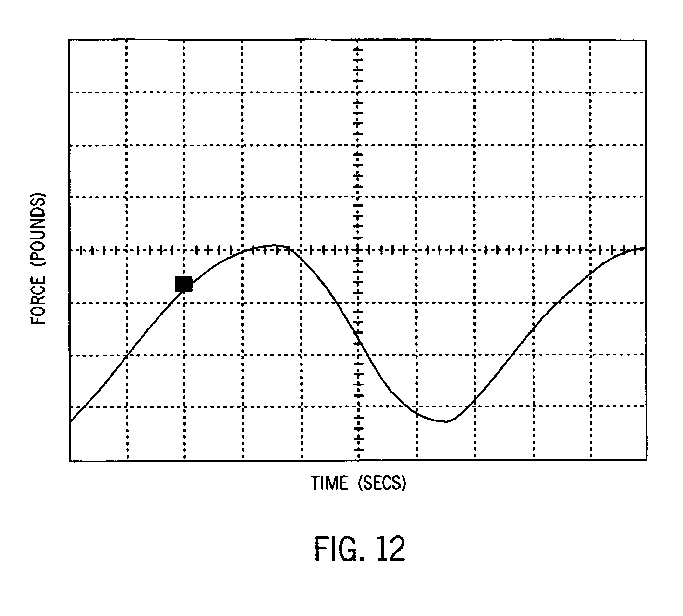

Referring to Fig. 12, an exemplary force curve is shown where the pumping element 38 moves in essentially a constant cyclic (sine-wave) motion, the pumping element 38 always has sufficient force available so that its speed is essentially independent of the force applied to the pumping element 38, and the outlet flow from pumping chamber 20 is not restricted. This curve was generated using an off-the-shelf force sensor available from Strain Measurement Devices, Inc. (SMD) of Meriden, Connecticut, U.S.A. under part number 2508-020 S420. Currently a customized force sensor available from SMD under part number BAT2656 is preferred for its smaller size and overload protection. However, one skilled in the art will appreciate that selection or design of a particular force sensor is a matter of routine design choice based on the size constraints and the desired functional sensor characteristics. The curve starts at Bottom Dead Center (BDC) with the pumping element 38 deflecting the pumping chamber 20 about 0.10 inches (2.54 mm) at this point. As the pumping element 38 moves into the cassette 12 (which is called the down-stroke or output stroke because fluid is flowing out of the cassette 12) the force builds to a maximum at Top Dead Center (TDC), shown about 1/3 of the way along the curve. The pumping element 38 then moves out of the cassette 12 (which is called the up-stroke or inlet stroke because fluid is flowing into to cassette 12) and the force drops off until it is minimum at BDC again (about 2/3 of the way along the curve). This curve then shows another BDC to TDC stroke for the final 1/3 of the curve (showing 1.5 full cycles of pumping element 38 movement). The position sensor 86 allows the pump 10 to sense when the pumping element 38 should be at BDC, TDC, or about 35° past BDC.

In a prior art diaphragm pump, the inlet valve would close, and the outlet valve would open at BDC to allow flow of fluid out of the cylinder during the output stroke. The pump 10 of present invention, however, is altered so that the outlet valve 24 does not open until the pumping element 38 has completed part of its output stroke (at roughly 35° past BDC). If the cassette 12 is full of liquid during the output stroke, the movement of the pumping element 38 into the cassette 12 with both the inlet and outlet valves 22 and 24 closed will result in a significant rise in the pressure of the liquid, and thus the resulting force on the pumping element 38. If the pumping chamber 20 of the cassette 12 is partly full of air, the pressure rise will be significantly lower, because air is much more compressible than liquid. Fig. 12 shows the force curve generated with air in the pumping chamber 20.

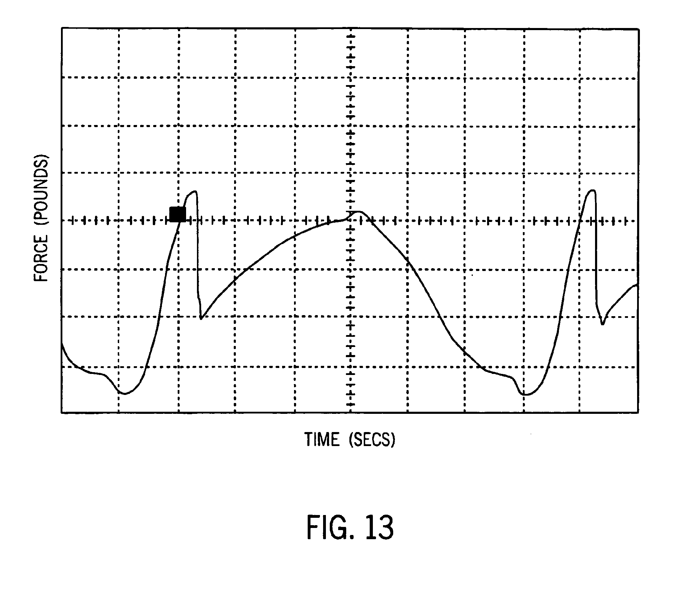

Referring to Fig. 13, a force curve generated with the cassette 12 full of liquid (note that BDC is now shifted slightly to the right and does not occur at the far left edge of the force trace) is shown. The large spikes result from the buildup of pressure in the cassette 12 exerting significant force onto the pumping element 38. When the outlet valve 24 opens (about one-fourth of the way along this curve) the pressure immediately drops, and the force exerted on the pumping element 38 now is only that resulting from the partly stretched diaphragm 21 of the pumping chamber 20. The pressure starts to rise just about 35° past BDC and the magnets 88 at that position provides a signal to allow the pump 10 to start taking data. By examining the force curve one can deduce whether the cassette 12 was full of liquid (i.e. a fluid stroke and exemplified by Fig. 13), or if the cassette 12 contained a significant amount of air (called an air stroke and exemplified by Fig. 12).

The processing unit 26 executes algorithm 32 to electronically examine the force curve and makes the determination of whether it is a fluid or air stroke. The result of this algorithm 32 produces a number called the Figure of Merit. If the Figure of Merit is above a threshold value of 450, the pump interprets this as a fluid stroke; below 450, as an air stroke. One of ordinary skill in the art will understand that these threshold values are predetermined empirically from experimental data, and will vary from pump model to pump model.

Pump 10 also has an automatic priming feature, to determine if the pump 10 has been automatically primed correctly or if it has failed to do so. When the cassette 12 is installed in the pump 10, the pump 10 primes the feeding tube set (not shown) attached to the cassette 12. The initial strokes of the pump 10 during priming will be recorded as air strokes because the liquid from the feeding tube set (not shown) has not yet reached the pumping chamber 20 in the cassette 12. If too many of these air strokes are recorded before fluid strokes are sensed, this is interpreted by the processing unit 26 as the pump 10 having failed to prime properly, and an appropriate alarm is provided to display 28 or otherwise. If the pump 10 records fluid strokes within the specified number of strokes, then the processing unit 26 knows that the cassette 12 has been primed successfully.

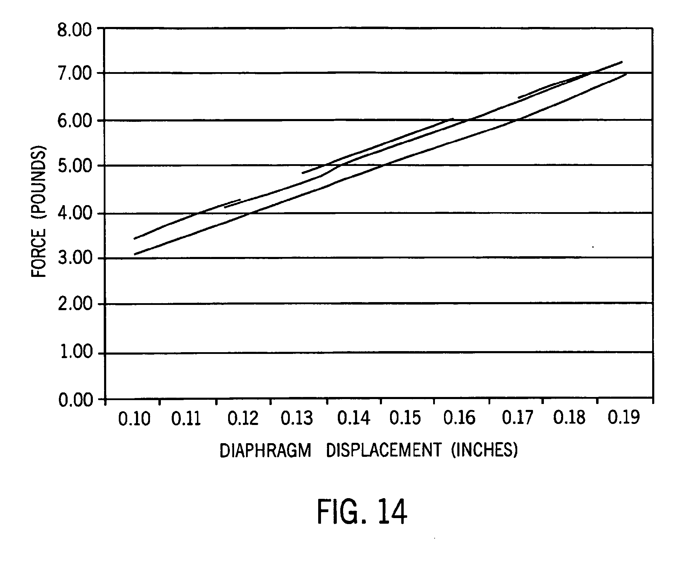

Referring to Fig. 14, pump 10 also determines if there is no cassette 12 in the pump 10. The diaphragm 21 of the pump chamber 20 of cassette 12 installed in the pump 10 will produce a certain amount of force on the pumping element 38. Fig. 14 shows the results of testing four cassettes to determine their force vs. deflection characteristics. About 0.10 inches (2.54 mm) of diaphragm 21 displacement is expected if a cassette 12 is installed in the pump 10. The force curve is examined to make sure that during a stroke, some minimum amount of force is registered on the pressure sensor 84. If this condition is not satisfied, the processing unit 26 interprets this to mean that no cassette 12 was installed in the pump 10 and an appropriate alarm is provided to display 28 or otherwise. A cassette out alarm may also be provided where the pressure sensor 84 fails to produce an output voltage.

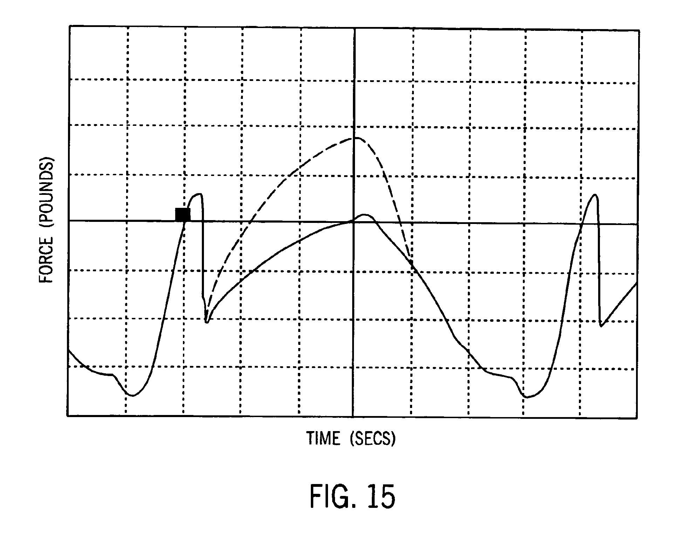

Referring to Fig. 15, by examining the force curve near TDC, the processing unit 26 can determine if the flow of fluid is blocked to the patient. The pressure on the pumping element 38 is the sum of the force exerted on the pumping element 38 by the diaphragm 21 (see Fig. 14) and the force on the pumping element 38 due to the pressure load. In normal operation the pressure is very low because the flow out of the cassette 12 is unimpeded. However, if the outlet flow is impeded by a significant restriction downstream of the outlet 18, the pressure will increase significantly near TDC as illustrated by the dashed line in Fig. 15. If that pressure is sufficiently large, the processing unit 26 generates a signal or alarm indicating that a flow blocked (occlusion) condition exists to display 28 or otherwise. It should be noted that the blocked flow alarm is sounded and/or displayed, even though some fluid flow may continue.

Once the pump 10 is in normal operation (i.e., pumping fluid) and the pressure sensor 84 registers four consecutive air strokes, the processing unit 26 interprets this condition as a bag empty condition. The processing unit 26 generates a bag empty alarm to display 28 or otherwise.

If one or both inlet and outlet slider assemblies 54 and 66 are not pressing the cassette 12 in such a manner as to cause a proper seal, the pressure spike will be suppressed. The suppressed pressure spike will result in a reduction in the calculated Figure of Merit. The processing unit 26 interprets this condition as an improper valve seal condition, and generates an alarm to display 28 or otherwise notifies the user of the pump 10. It is important to detect this condition because the pump 10 can over deliver under certain conditions if the inlet and outlet slider assemblies 54 and 66 do not produce a proper seal.

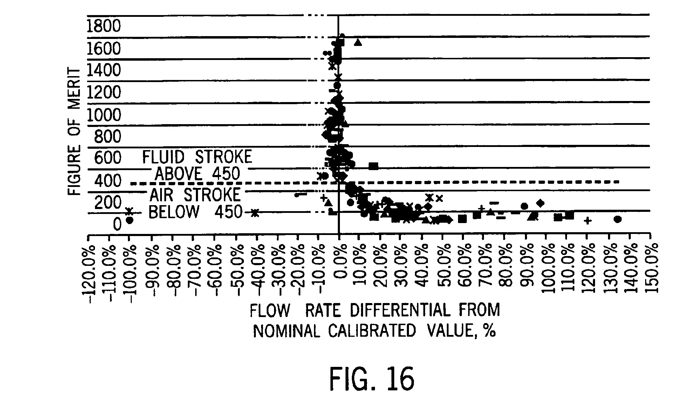

Referring to Fig. 16, test data taken with a wide range of cassettes and four different pumps is shown. This data shows that as long as the pump 10 is producing a Figure of Merit number that is interpreted by processing unit 26 to be a fluid stroke, the pump 10 will be producing a flow rate within about 10% of its calibrated value. Thus the pump 10 will not be over or under delivering a significant amount of fluid, so long as processing unit 26 determines fluid stroke conditions are met.

Whereas the invention has been shown and described in connection with the embodiments thereof, it will be understood that many modifications, substitutions, and additions may be made which are within the intended broad scope of the following claims. From the foregoing, it can be seen that the present invention accomplishes at least all of the stated objectives.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A medical pump (10) for use with a cassette (12) having a pumping chamber (20), comprising;
a pumping element (38) adapted to intermittently pressurize the
pumping chamber (20) during a pumping cycle; **characterized in that** it further comprises:
means (22, 24, 52, 64, 76) for closing the pumping chamber (20) to flow during at least
a portion of the pump cycle when the pumping chamber (20) is pressurized by the pumping element (38), and
a single pressure sensor (84) operatively connected to the pumping element (38) to detect the pressure exerted by the pumping element (38) on the pumping chamber (20).

2. A medical pump according to claim 1, comprising:
said pumping element (38) including a piston slider assembly (40) adapted to intermittently pressurize the pumping chamber (20) during a pumping cycle, the piston slider assembly (40) having a piston head (42) connected to a main body (44), the piston head (42) is adapted to contact the pumping chamber (20), and a pressure sensor (84) is connected to the pumping element (38) between the piston head (42) and the main body to detect the pressure exerted by the pumping element (38) on the pumping chamber (20).

3. The medical pump of claim 2, wherein the piston slider assembly (40) includes a bore (50) which passes through the main body (44) and provides a surface for transferring force from a pump motor (34) to the pumping element (38).

4. The medical pump of claim 2, wherein the piston slider assembly (40) includes slider elements (46) which permit the piston slider assembly (40) to be slidably associated with a pump housing (48).

5. A medical pump (10) according to claim 1, comprising:
said pumping element (38) adapted to intermittently pressurize the pumping chamber (20) during a pumping cycle;
an inlet control element (52) and an outlet control element (64) adapted to close the pumping chamber (20) to flow;
a camshaft (76) associated with the pumping element (38), inlet control element (52), and outlet control element (64) for closing the pumping chamber (20) to flow during at least a portion of the pump cycle when the pumping chamber (20) is pressurized by the pumping element (38); and
a pressure sensor (84) operatively connected to the pumping element (38) to detect the pressure exerted by the pumping element (38) on the pumping chamber (20).

6. The medical pump of any one of claims 1 and 2, further including a processing unit (26) in electronic communication with the pressure sensor (84), wherein the processing unit (26) processes pressure data from the pressure sensor (84) to determine the operating condition of the pump.

7. The medical pump of claim 6, wherein the operating condition determined is blocked fluid flow, no fluid in the line, no cassette associated with the pump, proper pump priming, or proper valve sealing.

8. The medical pump of claim 6, further including a position sensor (86) in electronic communication with the processing unit (26) and operatively associated with the pumping element (38) to detect the position of the pumping element (38), wherein the processing unit (26) processes position data from the position sensor (86) to associate the incoming pressure data with a particular portion of the pump cycle.

9. The medical pump of any one of claims 1 and 5, wherein the pumping element (38) includes a piston slider assembly (40) having a piston head (42) connected to a main body (44), the piston head (42) is adapted to contact the pumping chamber (20), and the single pressure sensor (84) is connected to the pumping element (38) between the piston head (42) and the main body (44).

10. The medical pump of any one of claims 2 and 5, wherein the pressure sensor (84) is the only pressure sensor included in the medical pump.

11. The medical pump of claim 10, wherein the pressure sensor (84) is a force sensor.

## Patentansprüche

1. Eine medizinische Pumpe (10) zur Verwendung mit einer Kassette (12), die eine Pumpenkammer (20) hat, folgendes umfassend:
ein Pumpen-Element (38), das ausgebildet ist, um die Pumpenkammer (20) während eines Pumpenzyklus intermittierend unter Druck zu setzen, **dadurch gekennzeichnet, dass** es weiter folgendes umfasst:
Mittel (22, 24, 52, 64, 76) zum Verschließen der Pumpenkammer (20) gegenüber Strömung während mindestens eines Abschnitts des Pumpenzyklus, wenn die Pumpenkammer (20) vom Pumpen-Element (38) unter Druck gesetzt wird, und
einen einzigen Drucksensor (84), der operativ mit dem Pumpen-Element (38) verbunden ist, um den Druck zu erfassen, der vom Pumpen-Element (38) auf die Pumpenkammer (20) ausgeübt wird.

2. Eine medizinische Pumpe gemäß Anspruch 1, die folgendes umfasst:
das Pumpen-Element (38), das einer Kolbenschieber-Gruppe (40) einschließt, die ausgebildet ist, um die Pumpenkammer (20) während eines Pumpenzyklus intermittierend unter Druck zu setzen, wobei die Kolbenschieber-Gruppe (40) einen Kolbenkopf (42) hat, der mit einem Hauptkörper (44) verbunden ist, wobei der Kolbenkopf (42) ist ausgebildet, um die Pumpenkammer (20) zu berühren, und ein Drucksensor (84) ist mit dem Pumpen-Element (38) zwischen dem Kolbenkopf (42) und dem Hauptkörper verbunden, um den Druck zu erfassen, der vom Pumpen-Element (38) auf die Pumpenkammer (20) ausgeübt wird.

3. Die medizinische Pumpe gemäß Anspruch 2, wobei die Kolbenschieber-Gruppe (40) eine Bohrung (50) einschließt, die sich durch den Hauptkörper (44) erstreckt und eine Oberfläche bereitstellt, um Kraft von einem Pumpenmotor (34) auf das Pumpen-Element (38) zu übertragen.

4. Die medizinische Pumpe gemäß Anspruch 2, wobei die Kolbenschieber-Gruppe (40) Schieberelemente (46) einschließt, die es der Kolbenschieber-Gruppe (40) ermöglichen, verschiebbar mit einem Pumpengehäuse (48) verbunden zu werden.

5. Eine medizinische Pumpe (10) gemäß Anspruch 1, die folgendes umfasst:
das Pumpen-Element (38), das ausgebildet ist, um die Pumpenkammer (20) während eines Pumpenzyklus intermittierend unter Druck zu setzen;
ein Einlass-Steuerelement (52) und ein Auslass-Steuerelement (64), ausgebildet, um die Pumpenkammer (20) gegenüber Strömung zu verschließen;
eine Nockenwelle (76), die mit dem Pumpen-Element (38), dem Einlass-Steuerelement (52) und den Auslass-Steuerelement (64) verknüpft ist, zum Verschließen der Pumpenkammer (20) gegenüber Strömung während zumindest eines Abschnitts des Pumpenzyklus, wenn die Pumpenkammer (20) vom Pumpen-Element (38) unter Druck gesetzt wird; und
einen Drucksensor (84), der operativ mit dem Pumpen-Element (38) verbunden ist, um den Druck zu erfassen, der vom Pumpen-Element (38) auf die Pumpenkammer (20) ausgeübt wird.

6. Die medizinische Pumpe gemäß irgendeinem der Ansprüche 1 und 2, weiter einen Prozessor (26) in elektronischer Kommunikation mit dem Drucksensor (84) einschließend, wobei der Prozessor (26) Druckdaten von Drucksensor (84) verarbeitet, um den Betriebszustand der Pumpe zu bestimmen.

7. Die medizinische Pumpe gemäß Anspruch 6, worin der ermittelte Betriebszustand blockierte Fluidströmung, kein Fluid in der Leitung, keine Kassette mit der Pumpe verknüpft, korrekte Pumpenansaugung oder korrekte Ventilabdichtung ist.

8. Die medizinische Pumpe gemäß Anspruch 6, die weiter einen Positionssensor (86) einschließt, der in elektronischer Kommunikation mit dem Prozessor (26) steht und operativ mit dem Pumpen-Element (38) verbunden ist, um die Position des Pumpen-Elements (38) zu ermitteln, wobei der Prozessor (26) Positionsdaten vom Positionssensor (86) verarbeitet, um die eingehenden Druckdaten mit einem bestimmten Abschnitt des Pumpenzyklus zu verknüpfen

9. Die medizinische Pumpe gemäß irgendeinem der Ansprüche 1 und 5, worin das Pumpen-Element (38) eine Kolbenschieber-Gruppe (40) einschließt, welche einen Kolbenkopf (42) hat, der mit einem Hauptkörper (44) verbunden ist, wobei der Kolbenkopf (42) ausgebildet ist, um die Pumpenkammer (20) zu berühren, und der einzige Drucksensor (84) ist mit dem Pumpen-Element (38) zwischen dem Kolbenkopf (42) und dem Hauptkörper (44) verbunden.

10. Die medizinische Pumpe gemäß irgendeinem der Ansprüche 2 und 5, worin der Drucksensor (84) der einzige in die medizinische Pumpe eingeschlossene Drucksensor ist.

11. Die medizinische Pumpe gemäß Anspruch 10, worin der Drucksensor (84) ein Kraftsensor ist.

## Revendications

1. Pompe médicale (10) pour une utilisation avec une cassette (12) comportant une chambre de pompage (20), comprenant :
un élément de pompage (38) adapté pour mettre sous pression de manière intermittente
la chambre de pompage (20) pendant un cycle de pompage ; **caractérisée en ce qu'**elle comprend en outre :
des moyens (22, 24, 52, 64, 76) pour fermer la chambre de pompage (20) à l'écoulement pendant au moins
une partie du cycle de pompe lorsque la chambre de pompage (20) est mise sous pression par l'élément de pompage (38), et
un capteur de pression unique (84) relié fonctionnellement à l'élément de pompage (38) pour détecter la pression exercée par l'élément de pompage (38) sur la chambre de pompage (20).

2. Pompe médicale selon la revendication 1, comprenant :
ledit élément de pompage (38) comprenant un ensemble coulisseau-piston (40) adapté pour mettre sous pression de manière intermittente la chambre de pompage (20) pendant un cycle de pompage, l'ensemble coulisseau-piston (40) comportant une tête de piston (42) reliée à un corps principal (44), la tête de piston (42) étant adaptée pour être en contact avec la chambre de pompage (20) et un capteur de pression (84) est relié à l'élément de pompage (38) entre la tête de piston (42) et le corps principal pour détecter la pression exercée par l'élément de pompage (38) sur la chambre de pompage (20).

3. Pompe médicale selon la revendication 2, dans laquelle l'ensemble coulisseau-piston (40) comprend un alésage (50) qui traverse le corps principal (44) et forme une surface pour le transfert de force d'un moteur de pompe (34) à l'élément de pompage (38).

4. Pompe médicale selon la revendication 2, dans laquelle l'ensemble coulisseau-piston (40) comprend des éléments de coulisseau (46) qui permettent à l'ensemble coulisseau-piston (40) d'être associé à coulissement à un logement de pompe (48).

5. Pompe médicale (10) selon la revendication 1, comprenant :
ledit élément de pompage (38) adapté pour mettre sous pression de manière intermittente la chambre de pompage (20) pendant un cycle de pompage ;
un élément de commande d'admission (52) et un élément de commande de sortie (64) adapté pour fermer la chambre de pompage (20) à l'écoulement ;
un arbre à came (76) associé à l'élément de pompage (38), l'élément de commande d'admission (52) et l'élément de commande de sortie (64) pour fermer la chambre de pompage (20) à l'écoulement pendant au moins une partie du cycle de pompe lorsque la chambre de pompage (20) est mise sous pression par l'élément de pompage (38) ; et
un capteur de pression (84) relié fonctionnellement à l'élément de pompage (38) pour détecter la pression exercée par l'élément de pompage (38) sur la chambre de pompage (20).

6. Pompe médicale selon l'une quelconque des revendications 1 et 2, comprenant en outre une unité de traitement (26) en communication électronique avec le capteur de pression (84), l'unité de traitement (26) traitant des données de pression provenant du capteur de pression (84) pour déterminer l'état opérationnel de la pompe.

7. Pompe médicale selon la revendication 6, dans laquelle l'état opérationnel déterminé est un écoulement de fluide bloqué, aucun fluide dans la conduite, aucune cassette associée à la pompe, un amorçage de pompe approprié ou une étanchéité de valve appropriée.

8. Pompe médicale selon la revendication 6, comprenant en outre un capteur de position (86) en communication électronique avec l'unité de traitement (26) et associé de manière fonctionnelle à l'élément de pompage (38) pour détecter la position de l'élément de pompage (38), laquelle unité de traitement (26) traite des données de position provenant du capteur de position (86) pour associer les données de pression entrantes à une partie particulière du cycle de pompe.

9. Pompe médicale selon l'une quelconque des revendications 1 et 5, dans laquelle l'élément de pompage (38) comprend un ensemble coulisseau-piston (40) comportant une tête de piston (42) reliée à un corps principal (44), la tête de piston (42) étant adaptée pour être en contact avec la chambre de pompage (20) et le capteur de pression unique (84) étant relié à l'élément de pompage (38) entre la tête de piston (42) et le corps principal (44).

10. Pompe médicale selon l'une quelconque des revendications 2 et 5, dans laquelle le capteur de pression (84) est le seul capteur de pression inclus dans la pompe médicale.

11. Pompe médicale selon la revendication 10, dans laquelle le capteur de pression (84) est un capteur de force.
